# EUROPÄISCHE PATENTSCHRIFT

(11) **EP 2 392 397 B1**
(45) Veröffentlichungstag und Bekanntmachung des Hinweises auf die Patenterteilung: **17.06.2015**
(21) Anmeldenummer: 11162266.8
(22) Anmeldetag: 13.04.2011
(51) Int. Cl.: B01F 11/00, B01F 15/00, B01F 5/06, G01N 33/49, G01N 27/07, G01N 27/22, G01N 11/02, B01F 13/00, G01N 11/00

(54) **Vorrichtung und Verfahren für Gerinnungstests von Blut**
device and method for testing of blood coagulation
dispositif et procédé pour les tests de coagulation du sang

(30) Priorität: 01.06.2010 DE 102010029555
(43) Veröffentlichungstag der Anmeldung: 07.12.2011
(73) Patentinhaber: Robert Bosch GmbH, 70442 Stuttgart (DE)
(72) Erfinder: Rothacher, Peter, 76646, Bruchsal (DE)

(56) Entgegenhaltungen:
- EP-A1- 1 702 207
- EP-A1- 2 112 508
- WO-A1-94/09895
- WO-A1-03/015923
- WO-A1-2005/105282
- WO-A1-2009/106906
- DE-A1- 19 710 358
- US-A- 3 656 716
- US-A- 3 700 408
- US-B1- 7 021 122

## Beschreibung

Die vorliegende Erfindung betrifft eine Vorrichtung und ein Verfahren zur Durchführung von Gerinnungstests von Blut.

### Stand der Technik

Insbesondere in mikrofluidischen Systemen ist das Mischen zweier Fluide oder das Auflösen eines Feststoffes in einem Fluid problematisch. In mikrofluidischen Systemen ist es in der Regel recht schwierig, konvektive Elemente zu integrieren beziehungsweise konvektive Effekte zu realisieren. Der Haupttransportmechanismus in derartig kleinen Dimensionen ist vielmehr die Diffusion, die im Falle von großen Molekülen sehr langsam sein kann, und zwar in manchen Beispielen deutlich mehr als vierzehn Stunden oder sogar einem Tag pro Zentimeter. Sollen zum Beispiel Feststoffe in einem Fluid in einer Mikrokammer aufgelöst werden, kann dieser Vorgang mehrere Stunden in Anspruch nehmen, bis eine homogene Verteilung des Feststoffs in der Lösung erreicht ist. Ebenso lange kann es dauern, bis ein gelöster Stoff nachdiffundiert, wenn er beispielsweise am Rand eines Mikrokompartiments abgeschieden wird.

Um die Probleme zu lösen, ist eine Reihe von Ansätzen bekannt. So wurden bei fluidischen Disks magnetische Beads in die Kompartimente gegeben. Diese magnetischen Beads können durch Magnete in Bewegung versetzt werden, wodurch Medien in dem Kompartiment mischbar sind. Ferner sind Ansätze bekannt, bei denen sich verschiedene Lösungen beim Durchströmen eines Kompartiments mischen, wie etwa Herringbone Mischer, Mäander Mischer oder split&recombine Mischer. Allgemein wird oftmals auf das Prinzip der Lamination für einen Mischvorgang zurückgegriffen. Darüber hinaus sind aktuierbare Kompartimente bekannt, bei denen sich Bereiche des Chips deformieren, und es gibt ferner Ansätze mit pneumatisch aktuierten Pumpen.

Eine weitere weit verbreitete Behandlung von Flüssigkeiten ist beispielsweise die Untersuchung von Blut, wie beispielsweise Gerinnungstests oder eine Untersuchung mit Bezug auf Thrombozytenfunktionsstörungen. Bei den hauptsächlich angewandten Routine-Gerinnungstests wird die Zeit von der Zugabe eines Aktivators bis zum Beginn der Thrombinbildung untersucht. Die Tests benötigen von der Blutabnahme bis zum Erhalt des Ergebnisses meist über eine Stunde. Sie berücksichtigen ferner nicht die Interaktion mit und die Funktion von Thrombozyten, geben keine Anhaltspunkte zur Qualität und/oder Festigkeit eines Clots und auch nicht zur Geschwindigkeit der Fibrinolyse. Somit sind derartige Tests im Management einer akuten Blutung von geringem Nutzen. Ferner stellen besonders Thrombozytenfunktionsstörungen neben den plasmatischen Gerinnungsstörungen einen wesentlichen Risikofaktor für Blutungskomplikationen dar.

Insbesondere bei Tests, die sich auf die Blutgerinnung beziehen, sind die Geräte vorwiegend tragbare batteriebetriebene Geräte, die zur Selbstkontrolle eingesetzt werden. Die Patienten nehmen dabei einen Tropfen ihres Blutes aus der Fingerbeere und führen ihre Tests wöchentlich selbst durch.

Es sind weiter Geräte für die Thromboelastographie bekannt. Hier wird ein oszillierend rotierender Stempel, aufgehängt an einer Torsionsfeder, in eine Küvette mit ca. 300µl Flüssigkeit umfassend Blut und Reagenzien getaucht und die Position des Stempels durch Reflexion an einem auf der Achse angebrachten Spiegel vermessen.

Zum Einsatz im Operationssaal sind einige Geräte mit unterschiedlichen Technologien bekannt. Relativ einfache Geräte sind die sogenannten Kugelkoagulometer. Bei diesen Geräten dreht sich eine Messküvette, in der sich eine Stahlkugel und ca. 300µl Flüssigkeit umfassend Blut und Reagenzien befinden, langsam um ihre Längsachse. Die Küvette ist in ihrem Messbereich schräg positioniert, weshalb die Stahlkugel aufgrund der Schwerkraft und der eigenen Trägheit am niedrigsten Punkt der Küvette liegen bleibt. Außerhalb der Küvette befindet sich ein Magnetsensor, der auf eine Lageänderung der Kugel reagiert. Ein sich bildender Gerinnungsfaden zieht die Kugel dabei aus dem Erfassungsbereich des Magnetsensors und stoppt einen zuvor gestarteten elektronischen Zeitmesser.

Ebenfalls mechanisch arbeitet ein anderes bekanntes System. Hier wird Blut mitsamt Aktivator in einen Spalt zwischen zwei kegelartigen Platten gegeben. Der entstehende Clot schrumpft, was zu einer Kraft führt, die die beiden Platten zusammenzieht. Diese Kraft wird über die Zeit gemessen, wodurch eine Aussage über die Thrombozytenfunktion möglich ist.

Weitere bekannte Systeme basieren auf der Impedanzaggregometrie.

DE 698 28 319 A2 beschreibt ferner ein Verfahren und Gerät zur Messung der Blut-Koagulation oder Lyse mit Hilfe von Viskositätsänderungen, wobei die Probe mit einer elektroaktiven Spezies kontaktiert wird und die Koagulation der Probe durch chemisches Reagieren mit einem Reagenz beschleunigt wird, wobei eine Viskositätsänderung nachweisbar ist. Die Viskosität der Probe wird gemessen und ein elektrisches Signal generiert, anhand dessen auf die Koagulation geschlossen werden kann.

Eine Integration von gerinnungsdiagnostischen Einheiten in extrakorpolare Zirkulationssysteme ist derzeit nicht bekannt.

Das US Patent 7,021,122 B1 lehrt eine Vorrichtung zur Blutuntersuchung mittels zweier Elektroden.

Die WO 03/015 923 A1 offenbart eine Vorrichtung gemäß dem Oberbegriff des Anspruch 1.

### Offenbarung der Erfindung

Gegenstand der Erfindung ist eine Vorrichtung zum Behandeln einer Flüssigkeit, umfassend wenigstens eine erste Kammer und eine zweite Kammer, wobei die erste und die zweite Kammer jeweils einen Flüssigkeitsbereich aufweisen, der durch ein flexibles Material jeweils von einem Fluidbereich getrennt ist, wobei die Flüssigkeitsbereiche der ersten und der zweiten Kammer fluidisch miteinander verbunden sind, und wobei die Flüssigkeitsbereiche jeweils einen mit einer Flüssigkeitsleitung verbundenen Flüssigkeitsanschluss und die Fluidbereiche jeweils einen mit einer Fluidleitung verbundenen Fluidanschluss aufweisen, wobei in den Flüssigkeitsleitungen oder an den Flüssigkeitsanschlüssen jeweils ein Ventil zum fluiddichten Verschließen der Flüssigkeitsleitungen angeordnet ist.

Unter dem Behandeln von Flüssigkeiten wird insbesondere ein Vermischen zweier oder einer Mehrzahl von Flüssigkeiten verstanden oder Untersuchen von Flüssigkeiten, wie etwa durch das Ausführen von (bio-) chemischen Reaktionen. Dazu zählen beispielsweise verschiedene medizinische Tests, wie etwa Gerinnungstests von Blut. Ferner ist eine Behandlung von Flüssigkeiten beziehungsweise einer Flüssigkeit das Auflösen von Feststoffen in einer Flüssigkeit.

Erfindungsgemäß ist es so möglich, die Fluidbereiche mit einem Fluid, wie etwa Druckluft, zu beaufschlagen, um so das flexible Material in Richtung des Flüssigkeitsbereichs zu deformieren. Durch eine abwechselnde Beaufschlagung der Fluidbereiche kann so erfindungsgemäß in den Kammern eine transversale Bewegung des Fluids oder der Fluide in den Flüssigkeitsbereichen entlang der Kammeroberfläche ausgelöst werden. Dadurch wird eine wellenartige Bewegung, also eine oszillierende Bewegung der Flüssigkeit zwischen den beiden Kammern verursacht. Es wird so eine schnellere Auflösung von Feststoffen beziehungsweise eine schnellere Abscheidung gelöster Feststoffe am Rand der Kammer, wie auch eine verbesserte Durchmischung zweier Flüssigkeiten bewirkt. Dabei muss weder die Kammer bewegt, noch Partikel eingebracht werden, noch das Fluid von außerhalb der Kammer eine Strömung erfahren.

Durch die Flexibilität des flexiblen Materials wird es so ferner möglich, im Verlaufe der Behandlung der Flüssigkeit eine weitere Substanz einzufügen, um Beispielsweise definierte Untersuchungen mit der Flüssigkeit durchzuführen.

Die Fertigungstechnologie lässt sich einfach mit membranbasierten Ventilen kombinieren. Bei der Durchmischung wird kein Hilfsmedium benötigt, so dass Verdünnungseffekte durch Transportflüssigkeiten vermieden werden können.

Mit der Verwendung der erfindungsgemäßen Vorrichtung lässt sich eine Viskositätsänderung im Blut leicht messen, womit sich der in der Medizin als "golden standard" etablierte Quicktest abbilden lässt. Gleichzeitig lässt sich in der gleichen Vorrichtung auch die Fibrinolyse bewerten. Es können ferner Thrombocytenfunktionsstörungen erkannt werden.

Die erfindungsgemäße Vorrichtung ist ferner sehr kostengünstig herzustellen, weshalb sie sich insbesondere als Wegwerfartikel, also als Artikel zur einmaligen Verwendung eignet.

In einer Anwendungsform lassen sich so beispielsweise auf einem Detektions-Array die Hybridisierung von DNA oder Antikörper-Antigen-Reaktionen stark beschleunigen.

Im Rahmen einer Ausführungsform ist die Vorrichtung ein Reaktor, insbesondere ein Mikroreaktor. Das bedeutet, dass die Vorrichtung beziehungsweise die Kammern einen abgrenzbaren beziehungsweise abgegrenzten Raum darstellen, in dem gezielte Vorgänge unter definierten Bedingungen ablaufen können. Im konkreten Fall wird der Raum abgegrenzt durch ein fluiddichtes Verschließen der Ventile. Sämtlicher Bauteile sollten inert sein und ablaufende Reaktionen nicht durch eventuelle Nebenreaktionen stören oder verfälschen. Dabei ist es essentiell, dass die Vorrichtung noch ihre vollständige Funktion ausüben kann, wie oben beschrieben.

Im Rahmen einer weiteren Ausführungsform sind der Flüssigkeitsbereich der ersten Kammer und der Flüssigkeitsbereich der zweiten Kammer durch eine Drossel miteinander verbunden. Dadurch ist die Bestimmung einer Veränderung der Viskosität der Flüssigkeit möglich.

Im Rahmen einer weiteren Ausführungsform ist zwischen der ersten Kammer und der zweiten Kammer eine dritte Kammer angeordnet, wobei die dritte Kammer einen Flüssigkeitsbereich aufweist, der von einem Fluidbereich durch ein flexibles Material getrennt ist, wobei der Flüssigkeitsbereich der dritten Kammer mit den Flüssigkeitsbereichen der ersten und der zweiten Kammer fluidisch verbunden ist. Durch das Vorsehen einer dritten Kammer können die erzielten Löse- beziehungsweise Mischvorgänge noch weiter verbessert werden. Dabei ist es besonders bevorzugt, wenn der Flüssigkeitsbereich der dritten Kammer mit den Flüssigkeitsbereichen der ersten und der zweiten Kammer jeweils durch Drosseln miteinander verbunden sind.

Erfindungsgemäß ist oberhalb und unterhalb wenigstens einer der ersten oder zweiten Kammer jeweils eine Flächenelektrode angeordnet. Dadurch kann auf einfache Art über eine kapazitive Untersuchung die Gerinnung eines Fluids, wie etwa Blut, oder die Fibrinolyse bestimmt werden. Da nur wenig Blut, insbesondere nur ein Tropfen aus der Fingerbeere, für die Füllung der beiden Kammern ausreicht, ist das Prinzip für tragbare Selbsttests geeignet.

Im Rahmen einer weiteren Ausführungsform sind in wenigstens einer der Flüssigkeitsbereiche interdigitierende Elektroden angeordnet. Mit dieser Ausführungsform kann konduktometrisch beispielsweise eine Thrombocytenaggregation beurteilt werden. Hierzu ist es vorteilhaft, wenn speziell beschichtete Elektroden, die eine Affinität zu den Pseudopodien der Thrombocyten haben, eingesetzt werden.

Im Rahmen einer weiteren Ausführungsform umfasst das flexible Material ein elastomeres Material, insbesondere ein thermoplastisches Elastomer. In diesem Fall kann die Erzeugung einer wellenartigen Bewegung und damit eine transversale Bewegung der Flüssigkeit besonders vorteilhaft gestaltet werden.

Gegenstand der Erfindung ist ferner ein Verfahren zur Durchführung von Gerinnungstests von Blut gemäß Anspruch 7.

Weitere Vorteile und vorteilhafte Ausgestaltungen der erfindungsgemäßen Gegenstände werden durch die Zeichnungen veranschaulicht und in der nachfolgenden Beschreibung erläutert. Dabei ist zu beachten, dass die Zeichnungen nur beschreibenden Charakter haben und nicht dazu gedacht sind, die Erfindung in irgendeiner Form einzuschränken. Es zeigen
- Fig. 1: eine schematische Schnittansicht der erfindungsgemäßen Vorrichtung von der Seite;
- Fig. 2: eine schematische Ansicht der erfindungsgemäßen Vorrichtung von oben;
- Fig. 3: eine schematische Schnittansicht einer Kammer einer Ausführungsform der erfindungsgemäßen Vorrichtung von der Seite;
- Fig. 4: eine schematische Draufsicht auf eine pneumatische Aktuationskammer für eine Verwendung mit der erfindungsgemäßen Vorrichtung;
- Fig. 5: eine schematische Teilansicht einer Ausführungsform der erfindungsgemäßen Vorrichtung mit Flächenelektroden;
- Fig. 6: eine schematische Teilansicht einer Ausführungsform der erfindungsgemäßen Vorrichtung mit interdigitierenden Elektroden.

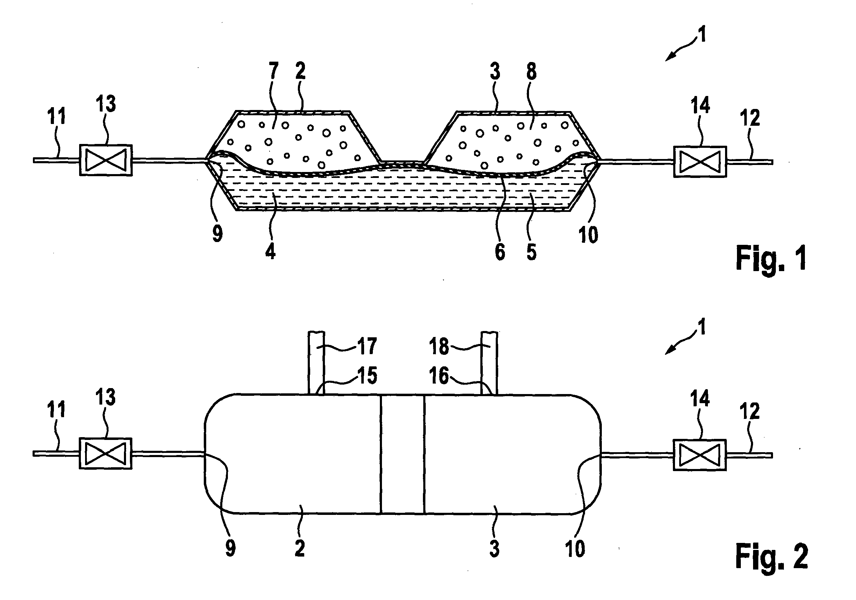

Figur 1 zeigt eine erfindungsgemäße Vorrichtung 1 zum Behandeln einer Flüssigkeit. Die Vorrichtung 1 ist geeignet, um beispielsweise das Vermischen zweier Flüssigkeiten zu verbessern, oder auch um Lösungsvorgänge zu beschleunigen. Darüber hinaus dient die erfindungsgemäße Vorrichtung zur Untersuchung von Flüssigkeiten.

Insbesondere ist die Vorrichtung 1 eine mikrofluidische Vorrichtung, jedoch nicht auf mikrofluidische Ausmaße begrenzt. Vielmehr ist eine erfindungsgemäße Vorrichtung 1 auch als makrofluidische Vorrichtung herstellbar und einsetzbar. Als mikrofluidische Vorrichtung wird hier eine Vorrichtung verstanden, die in wenigstens einer Dimension im Submilimeterbereich liegt.

Die erfindungsgemäße Vorrichtung 1 umfasst zwei Kammern, eine erste Kammer 2 und eine zweite Kammer 3. Die Kammern 2, 3 sind vorzugsweise aus einem Polymermaterial ausgebildet. Beispielsweise sind die Kammern aus einem Polymer ausgebildet, das metallisiert ist, etwa durch eine Metallisierung mit Kupfer, Nickel, Gold oder Mischungen hieraus. Die beiden Kammern 2, 3 weisen dabei jeweils einen Flüssigkeitsbereich beziehungsweise ein Flüssigkeitskompartiment auf. Daher ist in der ersten Kammer 2 ein erster Flüssigkeitsbereich 4 und in der zweiten Kammer 3 ein zweiter Flüssigkeitsbereich 5 angeordnet. Die Flüssigkeitsbereiche 4, 5 sind fluidisch miteinander verbunden, so dass ein Flüssigkeitsaustausch zwischen den Flüssigkeitsbereichen 4, 5 möglich ist.

Die Flüssigkeitsbereiche 4, 5 sind auf ihrer Oberseite durch ein flexibles Material 6 gedeckelt, beziehungsweise begrenzt. Das flexible Material 6 kann beispielsweise ein Elastomer, wie etwa ein thermoplastisches Elastomeren (TPE) umfassen. Beispielhafte thermoplastische Elastomere sind insbesondere auf Polyurethan basierende Polymere beziehungsweise Polurethan-Blends, da derartige Polymere besonders gut für ein Laserschweißen geeignet sind. Weitere mögliche Polmyere sind olefinbasierte Polymere, Copolyester, styrolbasierte Polymere oder auch Copolyamide. Das flexible Material 6 trennt die Flüssigkeitsbereiche 4, 5 dabei fluiddicht von den Fluidbereichen.

Dabei weisen die Kammern 2, 3 ferner neben den Flüssigkeitsbereichen 4, 5 jeweils einen Fluidbereich 7, 8 auf. Die Fluidbereiche 7, 8 sind dabei oberhalb des flexiblen Materials 6 angeordnet derart, dass die Flüssigkeitsbereiche 4, 5 von den Fluidbereichen 7, 8 durch das flexible Material 6 getrennt sind. Die Fluidbereiche 6, 7 sind dabei fluidisch von einander getrennt, so dass ein Gasaustausch zwischen dem Fluidbereich 6 der ersten Kammer 2 und dem Fluidbereich 7 der zweiten Kammer 3 nicht stattfinden kann. Erfindungsgemäß kann das flexible Material 6 einstückig ausgebildet sein, so dass die Flüssigkeitsbereiche 4, 5 von einem flexiblen Material begrenzt werden. Ferner kann jedoch für jeden der Flüssigkeitsbereiche ein eigenständiges flexibles Material vorgesehen sein.

Erfindungsgemäß ist es daher möglich, dass die Kammern 2, 3 vollständig getrennte Gebilde sind, bei denen die Flüssigkeitsbereiche 4, 5 miteinander verbunden sind. Diese Verbindung kann beispielsweise in Form einer oder mehrerer Drosseln realisiert sein. Eine Drossel ist hier ein Bereich, der gegenüber den Flüssigkeitsbereichen 4, 5, einen geringeren Umfang beziehungsweise Durchlass aufweist. In manchen Anwendungen ist es hierbei bevorzugt, dass in der Verbindung ein Ventil angeordnet ist. Alternativ ist es möglich, dass die beiden Flüssigkeitsbereiche 4, 5 Teil eines großen, ungetrennten Flüssigkeitsbereichs, wie etwa einer Wanne, sind, wobei die Größe der Kammern 2, 3 beziehungsweise die Kammern 2, 3 an sich durch die unabhängigen und von einander getrennten Fluidbereiche 7, 8 definiert sind.

Sind zwischen den Flüssigkeitsbereichen 4, 5 Verbindungen angeordnet, können diese fluidische Elemente oder Diffusoren umfassen, mit denen insbesondere die Fließrichtung der Flüssigkeit beeinflussbar ist. Als beispielhafte fluidische Elemente seinen hier Venturi-Düsen genannt. Dadurch ist auch, bei entsprechender Verbindung und entsprechender Druckbeaufschlagung der Fluidräume, ein kreisförmiges Fördern der Flüssigkeit möglich.

Die Kammern 2, 3 weisen an ihren Flüssigkeitsbereichen 4, 5 jeweils einen Flüssigkeitsanschluss 9, 10 auf, mit denen jeweils eine Flüssigkeitsleitung 11, 12 verbunden ist. In den Flüssigkeitsleitungen 11, 12 ist jeweils ein Ventil 13, 14 angeordnet, um die Fluidleitungen 11, 12 zu verschließen. Die Ventile 13, 14 können beispielsweise als pneumatische Ventile, wie etwa mikrofluidische Quetschventile, ausgebildet sein. Es sind jedoch jede Art von Ventilen, insbesondere mikrofluidische Ventile, möglich, die dem Fachmann geläufig sind.

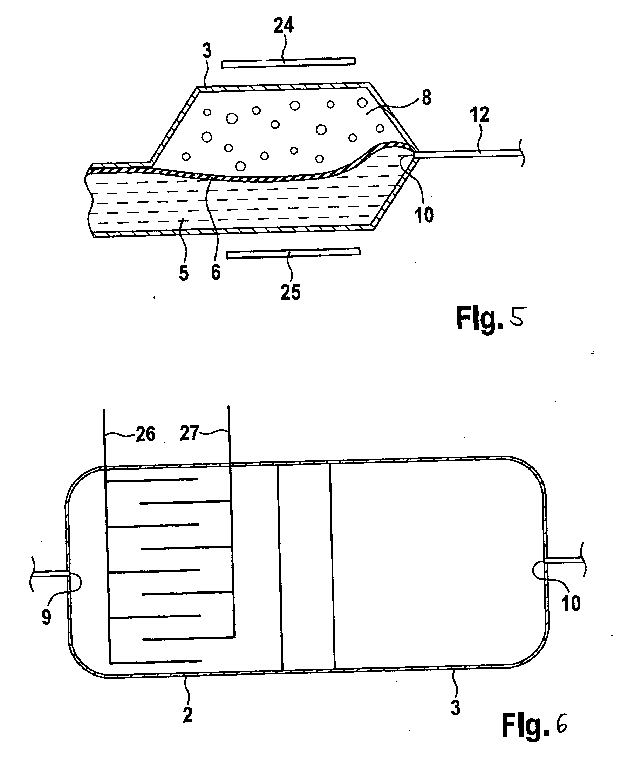

Darüber hinaus sind an den Fluidbereichen 7, 8 jeweils ein Fluidanschluss 15, 16 angeordnet, wie dies in Figur 2 zu erkennen ist. Verbunden mit den Fluidanschlüssen 15, 16 sind Fluidleitungen 17, 18. Die Fluidanschlüsse 15, 16 beziehungsweise die Fluidleitungen 17, 18 sind insbesondere mit einer Druckgasquelle verbunden, so dass die Fluidbereiche 7, 8 mit Druckgas beaufschlagbar sind. Die Fluidbereiche 7, 8 sind dann Gasbereiche. Besonders vorteilhaft sind die Druckgasquellen Druckluftquellen, wodurch eine Beaufschlagung mit Druckluft möglich ist. Beispielsweise kann dies eine Druckgasflasche beziehungsweise Druckluftflasche oder auch eine Druckluftversorgung wie ein Wandanschluss sein. Dabei kann die Druckgasquelle eine kleine Pumpe umfassen, so dass eine Versorgung mit Druckluft als tragbare Einheit realisierbar ist. Es ist ferner möglich, die Fluidanschlüsse 15, 16 über die Fluidleitungen 17, 18 mit einer pneumatischen Aktuationskammer zu verbinden, wie dies mit Bezug auf Figur 5 erläutert wird.

Dabei sind zweckmäßigerweise zwischen der Druckluftquelle und den Fluidbereichen 7,8 Ventile, wie etwa Pilotventile, angeordnet, um eine oszillierende Beaufschlagung der Fluidbereiche 7, 8 zu ermöglichen.

Um beispielsweise zwei Flüssigkeiten zu vermengen beziehungsweise mit einander zu vermischen, werden die Flüssigkeitsbereiche 4, 5 mit den zu vermischenden Flüssigkeiten, beispielsweise durch die Flüssigkeitsleitung 11 befüllt. Die in den Flüssigkeitsbereichen 4, 5 enthaltene Luft kann dabei durch den Flüssigkeitsanschluss 10 entweichen. Nach Befüllen der Flüssigkeitsbereiche 4, 5 werden die Ventile 13, 14 geschlossen, so dass die Flüssigkeitsbereiche 4, 5 einen abgeschlossenen Flüssigkeitsraum beziehungsweise einen Reaktor ausbilden.

Insbesondere durch abwechselnde Beaufschlagung der Fluidräume 7, 8 mit Druckgas, kann eine wellenartige Bewegung des elastomeren Materials 6 bewirkt werden. Dies kann auf einfache Weise durch eine antizyklische beziehungsweise oszillierende Druckbeaufschlagung der beiden Fluidräume geschehen, die dann pneumatische Kammern beziehungsweise pneumatische Kompartimente ausbilden. Für eine oszillierende Druckbeaufschlagung sind Pilotventile vorteilhaft, die in mit den Fluidanschlüssen verbundenen Fluidleitungen angeordnet sind. Ein oszillierendes Öffnen und Schließen dieser Ventile erfolgt dabei vorzugsweise mit einer Schaltzeit von 0,1 Sekunden. Dadurch wird die Flüssigkeit in den Kammern 2, 3 beziehungsweise in den Flüssigkeitsräumen 4, 5 entlang der Kammerwand transversal hin und her bewegt. Durch eine derartige Bewegung der Flüssigkeit beziehungsweise der Flüssigkeiten innerhalb der Flüssigkeitsräume 4, 5 kann eine gute Vermischung der Flüssigkeiten erzielt werden.

Ferner dient die vorliegende Vorrichtung zur Untersuchung einer Flüssigkeit, wie etwa der Gerinnungsuntersuchung von Blut. Hierzu werden die beiden Kammern 2, 3 beziehungsweise die Flüssigkeitsbereiche 4, 5 durch die Flüssigkeitsleitung 11 mit Blut befüllt. Insbesondere bei einer erfindungsgemäßen Vorrichtung 1 im Mikrofluidbereich, reicht hierzu ein Tropfen Blut aus. Dabei kann beispielsweise in der Flüssigkeitsleitung 11 ein Filter vorgesehen sein, der eine Abtrennung von Leuko- und Erytrocythen bewirkt. Die beiden Kammern 2, 3 werden befüllt und anschließend werden die Ventile 13, 14 geschlossen, so dass ein abgeschlossenes Volumen aus den beiden Flüssigkeitsbereichen 4, 5 entsteht. In einer der Kammern 2, 3 können ferner lyophilisierte Gerinnungsfaktoren, wie etwa Transfer-Faktoren, vorgelagert sein. Anschließend werden die beiden Fluidräume 7, 8 antizyklisch, insbesondere mit Druckluft, gefüllt.

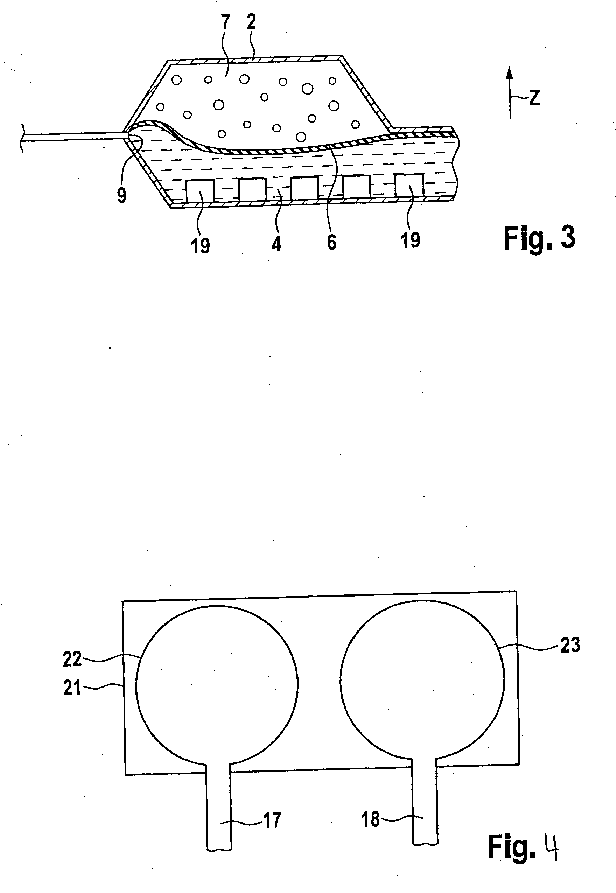

Hierzu kann beispielsweise eine membranbasierte Aktuationskammer 21 vorgesehen sein, wie dies in Figur 4 zu sehen ist. In dieser Aktuationskammer 21 sind Membranen 22, 23 vorgesehen, die beweglich in der Aktuationskammer 21 angeordnet sind. Durch eine beispielsweise durch einen Stößel bewirkte Bewegung wird ein unter- oder oberhalb der Membranen 22, 23 befindliches Volumen verkleinert, wodurch die Luft durch einen Pumpstoss beziehungsweise durch die Bewegung einer Membran 22, 23 durch die Fluidleitungen 17 beziehungsweise 18 in den entsprechenden Fluidraum 7, 8 gelangt. Dabei kann die Aktuationskammer 21 in einem Bauteil mit den Kammern 2, 3 ausgebildet sein, so dass sich diese Ausführungsform besonders gut für tragbare Systeme eignet.

Dadurch wird das flexible Material 6 in den Kammern 2, 3 zyklisch bewegt, wodurch eine wellenartige Bewegung des flexiblen Materials 6 erzeugt wird. Das in den Kammern 2, 3 befindliche Blut wird so ebenfalls in einer wellenartigen Bewegung von einer Kammer 2 in die andere Kammer 3 und umgekehrt gefördert. Auch der lyophilisierte Gerinnungsfaktor wird dabei aufgelöst, der die Gerinnungskaskade in Gang setzt. Verändert sich die Viskosität des Blutes bei der einsetzenden Gerinnung, kann pro Hub nicht mehr so viel Blut von einer Kammer 2 in die nächste Kammer 3, und umgekehrt, gefördert werden, so dass sich die Amplitude des flexiblen Materials verkleinert. Da Blut mit seinem Hauptbestandteil Wasser ein deutlich höheres Dipolmoment aufweist, als Luft, kann dies über eine kapazitive Auswertung ermittelt werden.

Erfindungsgemäß sind ober- und unterhalb weingstens einer der Kammern, beispielsweise der Kammer 3, Flächenelektroden 24, 25 vorgesehen sein. Eine Kammer 3 mit Flächenelektroden 24, 25 ist in Figur 5 gezeigt. Die Flächenelektroden 24, 25 sind über entsprechende Anschlüsse angesteuert beziehungsweise mit einer Auswerteeinheit verbunden. Diese Flächenelektroden 24, 25 können über die Menge an Blut in den Kammern und damit über die Menge an Dielektrikum die Kapazität messen und somit bestimmen, wieviel Blut zu jeder Zeit in der der entsprechenden Kammer ist. Dazu können vor einer Messung entsprechende Kalibrierungen vorgenommen werden, oder eine Auswertung erfolgt anhand der relativen Abnahme der Fördermenge zwischen den entsprechenden Kammern. Die Flächenelektroden 24, 25 können vorzugsweise die gleiche Größe beziehungsweise den gleichen Umfang aufweisen, wie die entsprechende Kammer, insbesondere die Kammer 3.

Auf diese Weise können durch die transportierte Menge an Blut Rückschlüsse auf die Viskosität des Bluts und damit auf seine Gerinnung gezogen werden.

Für die Bewertung einer Fibrinolysestörung kann der Test weiterbetrieben werden und eine gegebenenfalls einsetzende Verringerung der Blutviskosität kann ebenfalls kapazitiv durch eine wieder höher werdende Amplitude detektiert werden. Hierzu können beispielsweise zusätzliche Agentien in die Kammern 2, 3 eingeführt werden.

Ähnliche Untersuchungen können ferner durchgeführt werden, wenn beispielsweise in einer Drossel zwischen den beiden Kammern 2, 3 ein Durchflussmesser angeordnet ist. Auf diese Weise kann ebenfalls die Menge an gefördertem Blut und damit die Viskosität des Bluts ermittelt werden.

Um insbesondere eine Thrombocytenaggregation zu bewerten, können in einer der Kammern, beispielsweise in der Kammer 2 und zusätzlich zu den Flächenelektroden 24, 25 interdigitierende Elektroden 26, 27 angeordnet sein. Interdigitierende Elektroden 27, 28 sind hier ineinandergreifende Elektroden, etwa in Form von Kammelektroden, wie dies in Figur 6 zu sehen ist. Diese sind insbesondere in dem Flüssigkeitsbereich 4 angeordnet. Durch diese Elektroden 26, 27 kann die Thrombocytenaggregation konduktometrisch ausgewertet werden, da sich die Leitfähigkeit dadurch ändert, dass sich Thrombocyten auf der Elektrodenbeschichtung anlagern. Die Elektroden können dazu mit einer beispielsweise thrombin- und fibrin-Monomeren-haltigen Substanz, wie etwa einem Gel, beschichtet werden, das die Thrombocyten aktiviert und bindet. Auf diese Weise kann auch die Clot-Bildungsgeschwindigkeit und-Festigkeit, also die Bildungsgeschwindigkeit und -Festigkeit von Blutgerinseln beurteilt werden.

Diese Beschichtung kann ferner im Bereich der Drossel angeordnet werden. Wenn sich dort Thrombocyten ablagern, hat dies ebenfalls Einfluss auf de Amplitude des flexiblen Materials, Eine Differenzierung zwischen plasmatischer Gerinnung und Thrombocytenaggregation fällt dann zwar schwerer. Jedoch kann dieses Problem gelöst werden, wenn 3 Kammern mit zwei Drosseln vorgesehen sind, wobei dann nur eine Drossel beschichtet ist. Statt der interdigitierenden Elektroden 26, 27 können dann vorzugsweise zwei Kammern mit Flächenelektroden 24, 25 ausgestattet sein.

## Patentansprüche

1. Vorrichtung zur Durchführung von Gerinnungstests von Blut mit mindestens zwei Flächenelektroden, umfassend wenigstens eine erste Kammer (2) und eine zweite Kammer (3), wobei die erste (2) und die zweite (3) Kammer jeweils einen Flüssigkeitsbereich (4, 5) aufweisen, der jeweils durch ein flexibles Material (6) von einem Fluidbereich (7, 8) getrennt ist, wobei die Flüssigkeitsbereiche (4, 5) der ersten (2) und der zweiten (3) Kammer fluidisch miteinander verbunden sind, und wobei die Flüssigkeitsbereiche (4, 5) jeweils einen mit einer Flüssigkeitsleitung (10, 11) verbundenen Flüssigkeitsanschluss (9, 10) und die Fluidbereiche (7, 8) jeweils einen mit einer Fluidleitung (17, 18) verbundenen Fluidanschluss (15, 16) aufweisen, wobei in den Flüssigkeitsleitungen (10, 11) oder an den Flüssigkeitsanschlüssen (9, 10) jeweils ein Ventil (13, 14) zum fluiddichten Verschließen der Flüssigkeitsleitungen (10, 11) angeordnet ist, **dadurch gekennzeichnet, daß** oberhalb und unterhalb wenigstens einer der ersten (2) oder zweiten (3) Kammer jeweils eine Flächenelektrode (24, 25) angeordnet ist.

2. Vorrichtung nach Anspruch 1, **dadurch gekennzeichnet, dass** die Vorrichtung als ein Reaktor, insbesondere als ein Mikroreaktor, ausgebildet ist.

3. Vorrichtung nach Anspruch 1 oder 2, **dadurch gekennzeichnet, dass** der Flüssigkeitsbereich (4) der ersten Kammer (2) und der Flüssigkeitsbereich (5) der zweiten Kammer (3) durch eine Drossel miteinader verbunden sind.

4. Vorrichtung nach einem der Ansprüche 1 bis 3, **dadurch gekennzeichnet, dass** zwischen der ersten Kammer (2) und der zweiten Kammer (3) eine dritte Kammer angeordnet ist, wobei die dritte Kammer einen Flüssigkeitsbereich aufweist, der von einem Fluidbereich durch ein flexibles Material getrennt ist, wobei der Flüssigkeitsbereich der dritten Kammer mit den Flüssigkeitsbereichen (4, 5) der ersten (2) und der zweiten (3) Kammer fluidisch verbunden ist.

5. Vorrichtung nach einem der Ansprüche 1 bis 6, **dadurch gekennzeichnet, dass** in wenigstens einer der Flüssigkeitsbereiche (4, 5) interdigitierende Elektroden (26, 27) angeordnet sind.

6. Vorrichtung nach einem der Ansprüche 1 bis 9, **dadurch gekennzeichnet, dass** das flexible Material (6) ein elastomeres Material, insbesondere ein thermoplastisches Elastomer, umfasst.

7. Verfahren zur Durchführung von Gerinnungstests von Blut, wobei das Blut als Flüssigkeit in einen Flüssigkeitsbereich (4) einer ersten Kammer (2), der von einem ersten Fluidbereich (7) durch ein flexibles Material (6) getrennt ist und mit einem Flüssigkeitsbereich (5) einer zweiten Kammer (3), der von einem zweiten, von dem ersten Fluidbereich (7) getrennten, Fluidbereich (8) durch ein flexibles Material (6) getrennt ist, fluidisch verbunden ist, eingebracht wird und wobei das flexible Material (6) durch eine oszillierende Beaufschlagung der beiden Fluidbereiche (7, 8) mit Fluid derart verformt wird, dass das Blutt als Flüssigkeit in Bewegung versetzt wird, und wobei durch jeweils einer oberhalb und unterhalb wenigstens einer der ersten (2) oder zweiten (3) Kammer angeordneten Flächenelektroden (24, 25) eine Kapazität gemessen wird.

## Claims

1. Device for carrying out blood coagulation tests with at least two surface electrodes, comprising at least a first chamber (2) and a second chamber (3), wherein the first chamber (2) and the second chamber (3) each have a liquid region (4, 5) which is in each case separated from a fluid region (7, 8) by a flexible material (6), wherein the liquid regions (4, 5) of the first chamber (2) and second chamber (3) are fluidically connected to each other, and wherein the liquid regions (4, 5) each have a liquid connector (9, 10) connected to a liquid line (10, 11), and the fluid regions (7, 8) each have a fluid connector (15, 16) connected to a fluid line (17, 18), wherein a valve (13, 14) for closing the liquid lines (10, 11) in a fluid-tight manner is arranged respectively in the liquid lines (10, 11) or on the liquid connectors (9, 10), **characterized in that** a surface electrode (24, 25) is arranged respectively above and below at least one of first chamber (2) and second chamber (3).

2. Device according to Claim 1, **characterized in that** the device is designed as a reactor, in particular as a microreactor.

3. Device according to Claim 1 or 2, **characterized in that** the liquid region (4) of the first chamber (2) and the liquid region (5) of the second chamber (3) are connected to each other by a throttle.

4. Device according to one of Claims 1 to 3, **characterized in that** a third chamber is arranged between the first chamber (2) and the second chamber (3), wherein the third chamber has a liquid region which is separated from a fluid region by a flexible material, wherein the liquid region of the third chamber is fluidically connected to the liquid regions (4, 5) of the first chamber (2) and second chamber (3).

5. Device according to one of Claims 1 to 6, **characterized in that** interdigitated electrodes (26, 27) are arranged in at least one of the liquid regions (4, 5).

6. Device according to one of Claims 1 to 9, **characterized in that** the flexible material (6) comprises an elastomer material, in particular a thermoplastic elastomer.

7. Method for carrying out blood coagulation tests, wherein the blood is introduced as liquid into a liquid region (4) of a first chamber (2), which is separated from a first fluid region (7) by a flexible material (6) and is fluidically connected to a liquid region (5) of a second chamber (3), which is separated by a flexible material (6) from a second fluid region (8) separate from the first fluid region (7), and wherein the flexible material (6) is deformed, by an oscillating action of the fluid in the two fluid regions (7, 8), in such a way that the blood as liquid is set in motion, and wherein a capacitance is measured by surface electrodes (24, 25) arranged one above and one below at least one of the first chamber (2) and second chamber (3).

## Revendications

1. Dispositif pour mettre en oeuvre des tests de coagulation du sang, avec au moins deux électrodes planes, comprenant au moins une première chambre (2) et une deuxième chambre (3), la première (2) et la deuxième (3) chambre présentant chacune une zone de liquide (4, 5) qui est séparée à chaque fois par un matériau flexible (6) d'une zone de fluide (7, 8), les zones de liquide (4, 5) de la première (2) et de la deuxième (3) chambre étant connectées fluidiquement l'une à l'autre, et les zones de liquide (4, 5) présentant à chaque fois un raccord de liquide (9, 10) connecté à une conduite de liquide (10, 11) et les zones de fluide (7, 8) présentant à chaque fois un raccord de fluide (15, 16) connecté à une conduite de fluide (17, 18), une valve (13, 14) pour fermer les conduites de liquide (10, 11) de manière étanche aux fluides étant à chaque fois disposée dans les conduites de liquide (10, 11) ou au niveau des raccords de liquide (9, 10), **caractérisé en ce qu'**à chaque fois une électrode plane (24, 25) est disposée au-dessus et en dessous d'au moins l'une de la première (2) ou de la deuxième (3) chambre.

2. Dispositif selon la revendication 1, **caractérisé en ce que** le dispositif est réalisé sous forme de réacteur, en particulier sous forme de micro-réacteur.

3. Dispositif selon la revendication 1 ou 2, **caractérisé en ce que** la zone de liquide (4) de la première chambre (2) et la zone de liquide (5) de la deuxième chambre (3) sont connectées l'une à l'autre par un étranglement.

4. Dispositif selon l'une quelconque des revendications 1 à 3, **caractérisé en ce qu'**entre la première chambre (2) et la deuxième chambre (3) est disposée une troisième chambre, la troisième chambre présentant une zone de liquide qui est séparée d'une zone de fluide par un matériau flexible, la zone de liquide de la troisième chambre étant connectée fluidiquement aux zones de liquide (4, 5) de la première (2) et de la deuxième (3) chambre.

5. Dispositif selon l'une quelconque des revendications 1 à 6, **caractérisé en ce que** dans au moins l'une des zones de liquide (4, 5) sont disposées des électrodes (26, 27) s'emboîtant les unes dans les autres.

6. Dispositif selon l'une quelconque des revendications 1 à 9, **caractérisé en ce que** le matériau flexible (6) comprend un matériau élastomère, en particulier un élastomère thermoplastique.

7. Procédé pour mettre en oeuvre des tests de coagulation du sang, le sang étant introduit en tant que liquide dans une zone de liquide (4) d'une première chambre (2) qui est séparée d'une première zone de fluide (7) par un matériau flexible (6) et qui est connectée fluidiquement à une zone de liquide (5) d'une deuxième chambre (3) qui est séparée par un matériau flexible (6) d'une deuxième zone de fluide (8) séparée de la première zone de fluide (7), et le matériau flexible (6) étant déformé par une sollicitation en oscillation des deux zones de fluide (7, 8) par du fluide de telle sorte que le sang en tant que liquide soit mis en mouvement, et une capacité étant mesurée par des électrodes planes (24, 25) disposées respectivement au-dessus et en dessous d'au moins l'une de la première (2) ou de la deuxième (3) chambre.
